(19) Europäisches Patentamt
European Patent Office
Office européen des brevets

(11) Veröffentlichungsnummer: **0 306 866 B1**

# (12) EUROPÄISCHE PATENTSCHRIFT

(45) Veröffentlichungstag der Patentschrift: **03.03.93**

(51) Int. Cl.5: **C07D 487/04**, //(C07D487/04, 239:00,231:00)

(21) Anmeldenummer: **88114423.2**

(22) Anmeldetag: **03.09.88**

## (54) Verfahren zur Herstellung von Pyrazolo [5,1-b] chinazolonen.

(30) Priorität: **11.09.87 DE 3730535**

(43) Veröffentlichungstag der Anmeldung:
**15.03.89 Patentblatt 89/11**

(45) Bekanntmachung des Hinweises auf die Patenterteilung:
**03.03.93 Patentblatt 93/09**

(84) Benannte Vertragsstaaten:
**CH DE FR GB IT LI**

(56) Entgegenhaltungen:
**EP-A- 0 015 065**

**ANGEWANDTE CHEMIE, 74. Jahrgang, Nr. 21, 1962; K.H. MENZEL et al. "Synthesen und Reaktionen neuer ortho-kondensierter Pyrazoloverbindungen", Seiten 839-847**

(73) Patentinhaber: **BASF Aktiengesellschaft
Carl-Bosch-Strasse 38
W-6700 Ludwigshafen(DE)**

(72) Erfinder: **Kanter, Hartmut, Dr.
Niedererdstrasse 105
W-6700 Ludwigshafen(DE)**
Erfinder: **Ort, Burkhard, Dr.
Waldstrasse 63
W-6706 Wachenheim(DE)**

**EP 0 306 866 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Pyrazolo[5,1-b]chinazolonen durch Umsetzung von Isatosäureanhydriden mit 3-Methylpyrazol-5-on in Wasser als Reaktionsmedium bei einer Temperatur von 40 bis 150 ° C und einem Druck von 1 bis 5 bar.

Es ist bekannt, 2-Methylpyrazolo[5,1-b]chinazolon durch Umsetzung von Isatosäureanhydrid mit 3-Methylpyrazol-5-on in der Schmelze oder in einem hochsiedendem Lösungsmittel bei einer Temperatur von 200 bis 250 ° C durchzuführen (Angew. Chem. 74, 839 (1962)).

Aus J. Heterocycl. Chem. 18, 117 (1981), und J. Med. Chem. 24, 735 (1981), ist die Herstellung weiterer Pyrazolo[5,1-b]chinazolone bekannt. Als Ausgangsprodukte dienen ebenfalls Isatosäureanhydride und Pyrazolone. Die Umsetzung findet dabei in N,N-Dimethylformamid als Lösungsmittel in Gegenwart von Natriumhydrid als Base bei einer Temperatur von -10 bis 0 ° C statt.

Beide Verfahrensweisen sind im technischen Maßstab nicht ohne weiteres durchführbar. Zum einen bedarf es besonderer Maßnahmen, um die Umsetzung bei sehr hoher Temperatur in einer Schmelze oder in einem hochsiedenden Lösungsmittel vornehmen zu können; zum anderen ist die Verwendung von Natriumhydrid als Base nicht unproblematisch und verlangt einen hohen Sicherheitsaufwand.

Aufgabe der vorliegenden Erfindung war es, ein neues Verfahren zur Herstellung von Pyrazolo[5,1-b]-chinazolonen bereitzustellen, das von technisch leicht zugänglichen Ausgangsprodukten ausgeht und das die Zielprodukte ohne großen technischen Aufwand in guter Ausbeute liefert.

Es wurde nun gefunden, daß die Herstellung von Pyrazolo[5,1-b]chinazolonen der Formel I

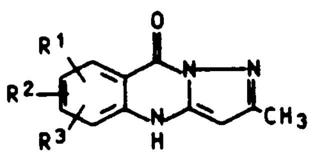

(I),

in der $R^1$, $R^2$ und $R^3$ gleich oder verschieden sind und unabhängig voneinander jeweils Wasserstoff, Fluor, Chlor oder Brom bedeuten, durch Umsetzung von Isatosäureanhydriden der Formel II

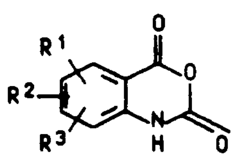

(II),

in der $R^1$, $R^2$ und $R^3$ jeweils die obengenannte Bedeutung besitzen, mit 3-Methylpyrazol-5-on vorteilhaft gelingt, wenn man die Umsetzung in Wasser als Reaktionsmedium bei einer Temperatur von 40 bis 150 ° C und bei einem Druck von 1 bis 5 bar durchführt.

Isatosäureanhydrid und 3-Methylpyrazol-5-on werden in der Regel im Molverhältnis 1,2:1 bis 0,6:1 umgesetzt. Beide Reaktionspartner können dabei jeweils auch als wäßrige Paste eingesetzt werden.

Es ist auch möglich, das 3-Methylpyrazol-5-on in Form seiner wäßrigen Syntheselösung, wie sie im allgemeinen bei der Umsetzung von Acetessigester mit Hydrazin anfällt, zu verwenden.

Eine bevorzugte Arbeitsweise besteht darin, das erfindungsgemäße Verfahren in Gegenwart von 5 bis 100 Gew.%, vorzugsweise 10 bis 25 Gew.%, jeweils bezogen auf das Isatosäureanhydrid II, eines Netzmittels durchzuführen.

Als Netzmittel kommen dabei an sich bekannte Tenside, wie sie z.B. in Ullmanns Encyklopädie der Technischen Chemie, 4. Auflage, Band 22, Seiten 467 bis 500, beschrieben sind, in Betracht. Beispielsweise seien Natriumsalze von Alkansulfonaten oder von Fettsäurekondensationsprodukten genannt.

Weiterhin bevorzugt ist eine Verfahrensweise, bei der man von Isatosäureanhydriden der Formel IIa

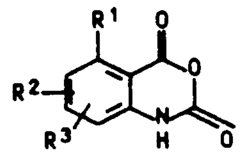

(IIa),

ausgeht, in der $R^1$ Fluor, Chlor oder Brom und $R^2$ und $R^3$ jeweils Wasserstoff bedeuten.

Zweckmäßig wird das erfindungsgemäße Verfahren so durchgeführt, daß man Wasser, 3-Methylpyrazol-5-on und gegebenenfalls Netzmittel vorlegt, unter Rühren auf eine Temperatur von 40 bis 100 °C, vorzugsweise 60 bis 80 °C erhitzt und das Isatosäureanhydrid langsam einträgt. Nach beendeter Kohlendioxid-Entwicklung, die die Bildung einer ringoffenen Zwischenverbindung der Formel III,

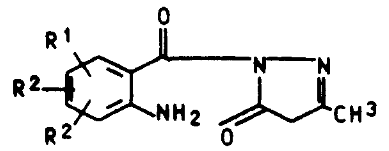

(III),

in der $R^1$, $R^2$ und $R^3$ jeweils die obengenannte Bedeutung besitzen, anzeigt, wird im allgemeinen 1 bis 10 Stunden bei der obengenannte Temperatur oder vorzugsweise bei einer Temperatur von 60 bis 150 °C, insbesondere 80 bis 130 °C nachgerührt.

Man arbeitet dabei in einem Druckbereich von 1 bis 5 bar, vorzugsweise 1 bis 3 bar. Die Reaktionstemperatur ist selbstverständlich vom Reaktionsdruck abhängig. Führt man das erfindungsgemäße Verfahren bei Normaldruck aus, so liegt die obere Grenze des Temperaturbereichs bei 100 °C. Bei Durchführung des Verfahrens bei höherem Druck liegt sie höher.

Nach beendeter Umsetzung wird das resultierende Pyrazolo[5,1-b]chinazolon der Formel I abgetrennt, mit Wasesr gewaschen und getrocknet.

Mittels des erfindungsgemäßen Verfahrens, das sowohl in kontinuierlicher als auch in diskontinuierlicher Arbeitsweise durchgeführt werden kann, können die Pyrazolo[5,1-b]chinazolone ohne großen technischen Aufwand und überraschenderweise unter Vermeidung der Anwendung von organischen Lösungsmitteln gewonnen werden.

Ein weiterer Vorteil des neuen Verfahrens besteht darin, daß jeweils gegebenenfalls unumgesetztes Ausgangsmaterial, Zwischenverbindung III sowie aus der Zwischenverbindung III durch Rückspaltung sich bildende Anthranilsäuren ohne Probleme vom Zielprodukt abgetrennt werden können. Die genannten Verbindungen gehen nämlich bei einem pH-Wert von 6,5 bis 8,5, vorzugsweise 7,0 bis 7,5, in warmem Wasser in Lösung. So genügt es üblicherweise schon, das Reaktionsgemisch durch Zusatz von wenig Base (z.B. Natron- oder Kalilauge) in den genannten pH-Bereich zu bringen und das Pyrazolo[5,1-b]chinazolon abzufiltrieren.

Bei den Pyrazolo[5,1-b]chinazolonen, die in guten Ausbeuten erhalten werden, handelt es sich um wertvolle Zwischenprodukte für die Synthese von Farbstoffen und Pigmenten.

Die folgenden Beispiele sollen die Erfindung näher erläutern.

Beispiel 1

Es wurden 300 g 3-Methylpyrazol-5-on und 50 g eines Netzmittels auf Basis des Natriumsalzes eines Fettsäurekondensationsproduktes in 6000 ml Wasser vorgelegt und unter Rühren auf 75 °C erhitzt. Man trug dann 500 g Isatosäureanhydrid innerhalb von 2 Stunden ein und rührte 10 Stunden nach. Anschließend wurde mit 10 gew.%iger Natronlauge ein pH-Wert von 8,5 eingestellt, 1 Stunde nachgerührt, abgesaugt, mit warmem Wasser gewaschen und getrocknet. Man erhielt 400 g eines farblosen Pulvers der Formel

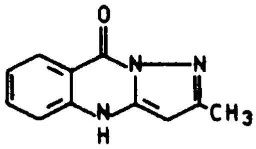

Beispiel 2

100 g 3-Methylpyrazol-5-on wurde in 600 ml Wasser vorgelegt, auf 70 °C erhitzt und 200 g 6-Chlorisatosäureanhydrid langsam eingetragen. Man rührte 1 Stunde nach, erhitzte auf 90 °C und rührte weitere 2 Stunden nach. Mit Natriumcarbonat wurde anschließend ein pH-Wert von 8 eingestellt, 1 Stunde gerührt, abgesaugt, mit warmem Wasser gewaschen und getrocknet. Man erhielt 200 g eines farblosen Pulvers der Formel

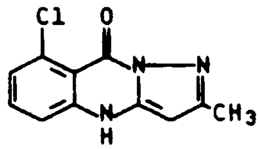

Beispiel 3

40 g 3-Methylpyrazol-5-on und 10 g eines Netzmittels auf Basis des Natriumsalzes eines Fettsäurekondensationsprodukts wurden in 650 ml Wasser vorgelegt, auf 70 °C erhitzt und 110 g 3,5,6-Trichlorisatosäureanhydrid langsam eingetragen. Man rührte 3 Stunden nach, erhitzte dann das Reaktionsgemisch auf 130 °C und rührte weitere 5 Stunden nach. Nach Abkühlen auf 80 °C wurde mit 5 gew.%iger Natronlauge ein pH-Wert von 8,5 eingestellt, 1 Stunde nachgerührt, abgesaugt, mit warmem Wasser gewaschen und getrocknet. Man erhielt 70 g eines hellen Pulvers der Formel

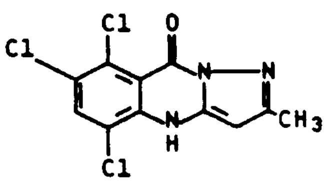

Die in der folgenden Tabelle aufgeführten Verbindungen wurden in analoger Weise erhalten. Reaktionstemperatur und Ausbeute sind jeweils angegeben.

| Beispiel Nr. | Formel | Reaktions-temp. [°C] | Ausbeute [%] |
|---|---|---|---|
| 4 | 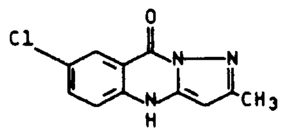 | 70/130 | 65 |
| 5 | 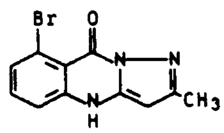 | 70/90 | 87 |
| 6 | 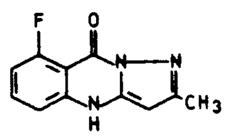 | 70/90 | 83 |
| 7 | 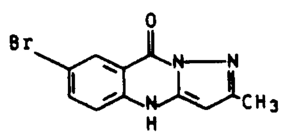 | 80/120 | 53 |
| 8 | 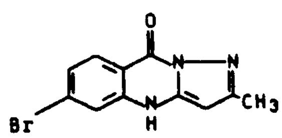 | 80/120 | 67 |
| 9 | 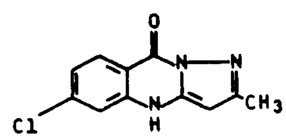 | 80/120 | 73 |
| 10 | 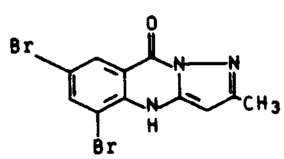 | 80/130 | 63 |

| Beispiel Nr. | Formel | Reaktions-temp. [°C] | Ausbeute [%] |
|---|---|---|---|
| 11 | 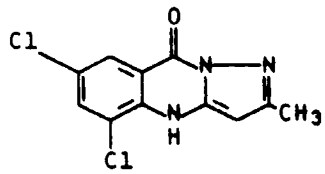 | 80/130 | 61 |
| 12 | 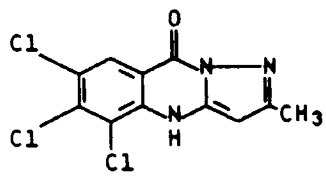 | 80/120 | 57 |
| 13 | 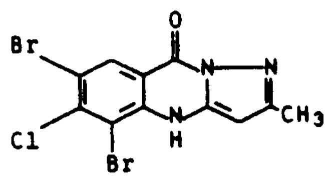 | 80/110 | 73 |

Beispiel 14

104 g Acetessigsäuremethylester wurden in 40 ml Wasser vorgelegt. Dazu ließ man innerhalb 1 Stunde 48 g Hydrazinhydrat bei 35 ° C bis 40 ° C zulaufen. Es wurde 2 Stunden bei 35 ° C bis 40 ° C nachgerührt, weitere 7 g Acetessigsäuremethylester zugesetzt und weitere 2 Stunden bei 55 ° C nachgerührt. Anschließend wurde mit 10 gew.%iger Natronlauge ein pH-Wert von 7 eingestellt. Man verdünnte mit 2 l Wasser, erwärmte auf 80 ° C und trug innerhalb von 30 Minuten 198 g 6-Chlorisatosäureanhydrid ein. Dann rührte man 5 Stunden bei 80 ° C und 1 Stunde bei 95 ° C nach und stellte mit 10 gew.%iger Natronlauge einen pH-Wert von 8 ein. Das Reaktionsprodukt wurde abgesaugt, mit warmem Wasser gewaschen und getrocknet. Man erhielt 190 g eines farblosen Pulvers der Formel

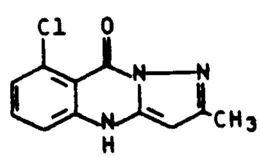

**Patentansprüche**

**1.** Verfahren zur Herstellung von Pyrazolo[5,1-b]chinazolonen der Formel I

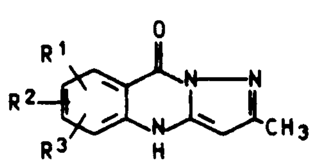 (I),

in der $R^1$, $R^2$ und $R^3$ gleich oder verschieden sind und unabhängig voneinander jeweils Wasserstoff, Fluor, Chlor oder Brom bedeuten, durch Umsetzung von Isatosäureanhydriden der Formel II

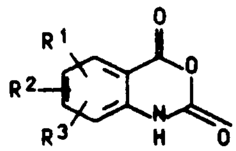
(II),

in der $R^1$, $R^2$ und $R^3$ jeweils die obengenannte Bedeutung besitzen, mit 3-Methylpyrazol-5-on, dadurch gekennzeichnet, daß man die Umsetzung in Wasser als Reaktionsmedium bei einer Temperatur von 40 bis 150 °C und bei einem Druck von 1 bis 5 bar durchführt.

2. Verfahren gemäß Anspruch 1, dadurch gekennzeichnet, daß man die Umsetzung in Gegenwart eines Netzmittels durchführt.

3. Verfahren gemäß Anspruch 1, dadurch gekennzeichnet, daß man die Umsetzung mit Isatosäureanhydriden der Formel IIa

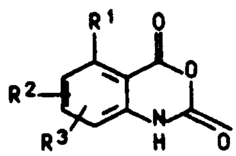
(IIa)

durchführt, in der $R^1$ Fluor, Chlor oder Brom und $R^2$ und $R^3$ jeweils Wasserstoff bedeuten.

## Claims

1. A process for preparing a pyrazolo[5,1-b]quinazolone of the formula I

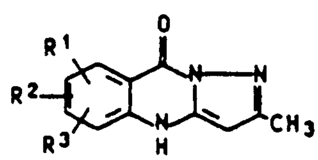
(I)

where $R^1$, $R^2$ and $R^3$ are identical or different and each is independently of the others hydrogen, fluorine, chlorine or bromine, by reacting an isatoic anhydride of the formula II

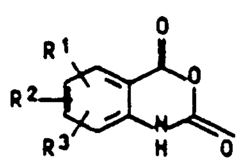
(II)

where $R^1$, $R^2$ and $R^3$ are each as defined above, with 3-methylpyrazol-5-one, which comprises performing the reaction in water as reaction medium at from 40 to 150 °C under from 1 to 5 bar.

2. A process as claimed in claim 1, wherein the reaction is carried out in the presence of a wetting agent.

3. A process as claimed in claim 1, wherein the reaction is carried out with an isatoic anhydride of the formula IIa

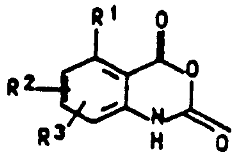

(IIa)

where $R^1$ is fluorine, chlorine or bromine and $R^2$ and $R^3$ are each hydrogen.

## Revendications

1. Procédé de préparation de pyrazolo[5,1-b]quinazolones de formule I

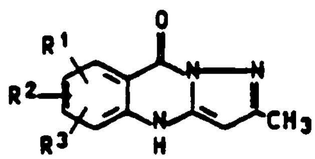

(I),

dans laquelle $R^1$, $R^2$ et $R^3$ sont identiques ou différents et représentent chacun, indépendamment les uns des autres, un atome d'hydrogène, de fluor, de chlore ou de brome, par réaction d'anhydrides isatoïques de formule II

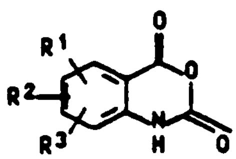

(II),

dans laquelle $R^1$, $R^2$ et $R^3$ ont chacun les significations sus-indiquées, avec de la 3-méthylpyrazole-5-one, caractérisé en ce qu'on conduit la réaction dans de l'eau servant de milieu réactionnel, à une température de 40 à 150 ° C et sous une pression de 1 à 5 bar.

2. Procédé selon la revendication 1, caractérisé en ce qu'on conduit la réaction en présence d'un agent dispersant.

3. Procédé selon la revendication 1, caractérisé en ce qu'on effectue la réaction avec des anhydrides isatoïques de formule IIa

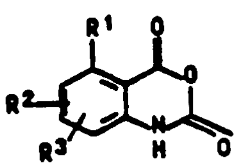

(IIa)

dans laquelle $R^1$ représente un atome de fluor, de chlore ou de brome et $R^2$ et $R^3$ représentent chacun un atome d'hydrogène.